**Europäisches Patentamt**

**European Patent Office**

**Office européen des brevets**

(11) Publication number: **0 336 693 B1**

## EUROPEAN PATENT SPECIFICATION

(45) Date of publication of patent specification: **30.11.94**

(21) Application number: **89303293.8**

(22) Date of filing: **04.04.89**

(51) Int. Cl.⁵: **A61K 39/395**, //(A61K39/395, 37:54)

The file contains technical information submitted after the application was filed and not included in this specification

(54) **Acceleration of clot lysis.**

(30) Priority: **04.04.88 US 177222**

(43) Date of publication of application:
**11.10.89 Bulletin 89/41**

(45) Publication of the grant of the patent:
**30.11.94 Bulletin 94/48**

(84) Designated Contracting States:
**AT BE CH DE ES FR GB GR IT LI LU NL SE**

(56) References cited:
**WO-A-87/06836**

**THROMBOSIS RESEARCH, vol. 36, 1984; T. KUMADA et al., pp. 153-163&NUM;**

(73) Proprietor: **THE GENERAL HOSPITAL CORPO-RATION**
**Fruit Street**
**Boston, Massachusetts 02114 (US)**

(72) Inventor: **Reed, Guy L.**
**19 Garden Street**
**Cambridge, MA 02138 (US)**
Inventor: **Matsueda, Gary**
**3 Standish Lane**
**Winchester, MA 01890 (US)**
Inventor: **Haber, Edgar**
**83 Ridgeway Road**
**Weston, MA 02193 (US)**

(74) Representative: **Wright, Simon Mark et al**
**Kilburn & Strode**
**30 John Street**
**London WC1N 2DD (GB)**

# Description

## FIELD OF THE INVENTION:

The present invention relates to a treatment for myocardial infarction and blood clots within a patient, and more specifically to a therapy which enhances clot lysis comprising administering an antibody directed to alpha-2-antiplasmin. The invention also relates to a treatment for enhancing clot lysis comprising administering an antibody directed toward alpha-2-antiplasmin together with a thrombolytic agent.

## BACKGROUND OF THE INVENTION

The initiating event of many myocardial infarctions (heart attacks) is the hemorrhage into atherosclerotic plaque. Such hemorrhage often results in the formation of a thrombus (or blood clot) in the coronary artery which supplies the infarct zone (i.e., an area of coagulation necrosis which results from an obstruction of blood circulation). This thrombus is composed of a combination of fibrin and blood platelets. The formation of a fibrin-platelet clot has serious clinical ramifications. The degree and duration of the occlusion caused by the fibrin-platelet clot determines the mass of the infarct zone and the extent of damage.

## A. Treatment for Myocardial Infraction

The primary goal of current treatment for myocardial infarction involves the rapid dissolution of the occluding thrombus and the restoration of blood flow ("reperfusion"). In order to be effective, a successful therapy must be capable of discriminating between a fibrin-platelet clot and the fibrin precursor, fibrinogen. The use of an agent which fails to exhibit such specificity may increase the risk of general hemorrhage to the patient. A successful therapy must be capable of sustained effect so that reformation of the clot does not occur after the cessation of therapy. If the fibrin-platelet clot is able to reform, then the affected artery may become reoccluded.

The formation of fibrin-platelet clots in other parts of the circulatory system may be partially prevented through the use of anti-coagulants (such as heparin). Unfortunately, heparin has not been found to be universally effective in preventing reocclusion in myocardial infarction victims in which the degree of blood vessel occlusion (the degree of "stenosis") is greater than or equal to 70%, particularly in those patients with severe residual coronary stenosis.

If an individual has formed a fibrin-platelet clot prior to the availability of medical assistance, the clot may be dissolved through the use of thrombolytic agents. A thrombolytic agent is a medicament capable of lysing the fibrin-platelet thrombus, and thereby permitting blood to again flow through the affected blood vessel. Such agents include streptokinase, prourokinase, urokinase, and tissue-type plasminogen activator (Ganz, W. et al., J. Amer. Coll. Cardiol. 1:1247-1253 (1983); Rentrop, K.P. et al., Amer. J. Cardiol. 54:29E-31E (1984); Gold, H.K. et al., Amer. J. Cardiol. 53:122C-125C (1984)).

Treatment with thrombolytic agents can often successfully restore coronary blood flow rapidly enough to interrupt myocardial infarction. Unfortunately, the dissolved fibrin-platelet clot has been found to reform after cessation of such thrombolytic therapy in a substantial number of patients. This reformation may result in the reocclusion of the affected blood vessels, and is, therefore, of substantial concern (Gold, H.K. et al., Amer. J. Cardiol. 53:122C-125C (1984); Gold, H.K. et al., Circulation 68:I-50-I-54 (1983)). Thus, although streptokinase treatment has been found to be successful in dissolving fibrin clots in approximately 85% of studied cases, reocclusion of the affected vessels has been found to occur in approximately 25% of the patients examined. (Gold, H.K., et al., Circulation, 68:I50-I54 (1983)).

Tissue-type plasminogen activator (t-PA) is considered to be a more desirable thrombolytic agent than either streptokinase or urokinase because it displays greater (though not absolute) specificity for fibrin than does either of these agents (Verstrate, M., et al., Lancet, 1:142 (1985)). Tissue-type plasminogen activator (t-PA) is a clot-specific thrombolytic agent with a rapid disposition rate from plasma. Tissue-type plasminogen activator (t-PA) has been found to be an effective thrombolytic agent in patients with acute myocardial infarction, producing coronary reflow (i.e., decreasing stenosis) in 45-75 minutes in approximately 70% of patients studied (Gold, H.K. et al., Circulation 73:347-352 (1986)).

Tissue-type plasminogen activator is administered at an infusion at a rate of approximately 1-2 mg/kg patient weight-/90 minutes. Because t-PA at high concentration is capable of breaking down fibrinogen, the use of higher dosages has been associated with an increased potential of general hemorrhage. Increased t-PA dosages have not been found to uniformly increase the rate of clot dissolution.

The benefit of employing t-PA significantly offset by the spontaneous rate of acute reocclusion which follows the cessation of t-PA therapy. Gold, H.K. and coworkers have found that cessation of t-PA therapy resulted in reocclusion of affected blood vessels in approximately 45% of patients studied (Circulation 73:347-352 (1986)). Increased t-PA dosages have not been found to decrease the tendency for coronary artery reocclusion. Significantly, the possibility of thrombin clot reformation is closely related to the degree of residual coronary stenosis (i.e., the extent of blood vessel blockage). Thus, reocclusion is more probable in individuals in which high grade stenosis (i.e., greater than 70% quantitative stenosis or greater than 80% non-quantitative stenosis) has occurred. The reocclusion of blood vessels has been found to be inhibited by continued infusion of t-PA (Gold, H.K. et al., Circulation 73:347-352 (1986)). Unfortunately, the relatively short biological half-life of t-PA and the potential for increasing the tendency for severe bleeding in some patients may make continued infusion of t-PA impractical for many heart attack victims.

In summary, clinical investigations have shown that the dissolved thrombus frequently reforms following the cessation of t-PA infusion (Gold, H.K. et al., Circulation 73:347-352 (1986)), but that the frequency of such reocclusion can be minimized by providing a second ("maintenance") t-PA infusion of a substantially lower dose but for a substantially longer period. Heparin is currently recognized as the appropriate concommitant therapy for patients receiving such a maintenance infusion. The treatment of coronary artery thrombosis clotting) with t-PA requires, therefore, a continuous infusion at a high rate in order to obtain rapid reperfusion, and a maintenance infusion at a lower dose to prevent reocclusion in patients with high grade residual stenosis.

B. Mechanism of Fibrin Clot Formation

Clots are composed of both fibrin and blood platelets in various ratios. The fundamental reaction in blood clotting involves the conversion of a soluble plasma protein (fibrinogen) into insoluble fibrin. The conversion of fibrinogen into fibrin is catalyzed by the enzyme, thrombin, which is a serine protease. The general mechanism of blood clot formation is reviewed by Ganong, W.F. (In: Review of Medical Physiology, 9th ed., Lange, Los Altos, CA, pp. 411-414 (1979)). Platelets are disk-shaped structures present in blood. They contribute to clot formation by both their incorporation with fibrin into an insoluble mass and by their enhancement of the rate of fibrinogen to fibrin conversion. Platelets contribute to clot formation in myocardial infarction and are a major component of clots that reocclude coronary arteries that have been reperfused by treatment with a thrombolytic agent. The formation of the platelet aggregate depends upon an interaction between fibrinogen (and perhaps von Willebrand's factor or fibronectin) and a receptor molecule present on the surface of platelets. This platelet fibrinogen receptor has been found to be a complex of two membrane glycoproteins, termed GPIIb and GPIIIa (Nachman, R.L. et al., J. Clin. Invest. 69:263-269 (1982); Coller, B.S. et al., J. Clin. Invest. 72:325-338 (1983)). The specific role of the GPIIb/GPIIIa receptor complex was elucidated by Coller, B.S. and coworkers through their isolation of a murine monoclonal antibody (known as monoclonal antibody 10E5) found to be capable of binding to glycoproteins IIb and IIIa, and of completely blocking the binding of fibrinogen to platelets. In order to avoid potential complications due to the possibility that the monoclonal antibody's Fc fragment region might inhibit aggregation non-specifically, Coller, et al. used the F(ab')$_2$ fragment of the 10E5 antibody in their experiments. (Coller, B.S. et al., J. Clin. Invest. 72:325-338 (1983)). The F(ab')$_2$ fragment of an antibody includes only those regions of the antibody which are responsible for the antibody's specificity and antigen-binding capacity. The nature of F(ab')$_2$ fragments and procedures for their preparation are disclosed by Eisen, H.N. (In: Microbiology, 3rd ed., Davis, B.D. et al., Harper & Row, N.Y., pp. 342-349 (1980)).

An additional monoclonal antibody (designated 7E3) was found to block the binding of fibrinogen to platelets, and to bind to GPIIb/GPIIIa (Coller, B.S., J. Clin. Invest., 76:101-108 (1985)). This monoclonal antibody differed from antibody 10E5 in that it bound much more rapidly to activated platelets than to unactivated platelets and was capable of binding to canine as well as human platelets (Coller, B.S., J. Clin. Invest. 76:101-108 (1985); Coller, B.S. et al., J. Lab. Clin. Med., 107:384-392 (1986); both of which references are incorporated by reference herein). The F(ab')$_2$ fragments of monoclonal antibody 7E3 were found to be capable of interfering with platelet aggregation, thus suggesting a potential therapeutic use in the treatment of thrombotic disease (Coller, B.S. et al., Blood 66:1456-1459 (1985)). The F(ab')$_2$ fragment of monoclonal antibody 7E3 was also found to be effective in blocking the accumulation of multiple layers of platelets without producing an unacceptable risk of hemorrhage, thus suggesting a potential use in avoiding the total occlusion of blood vessels which may occur in myocardial infarction and stroke (Coller, B.S. et al., Blood 66:1456-1459 (1985)).

C. Mechanism of Clot Lysis and Natural Inhibition Thereof

Clot lysis is mediated by plasmin in vivo. Under natural conditions, plasminogen is converted to plasmin by tissue plasminogen activator (t-PA). Activation occurs on the fibrin surface, thus confining proteolytic activity to the appropriate site. After plasmin is set free into the circulation, it is rapidly combined with natural inhibitors. Inactivation of plasmin is the final and necessary step in the process of protecting against undesirable proteolysis. Such plasmin inhibitors include alpha-2-antiplasmin, alpha-2-macroglobulin and alpha-1-antitrypsin, all glycoproteins. Alpha-2-antiplasmin has a much higher affinity for plasmin than alpha-2-macroglobulin and binds specifically to plasmin in a 1:1 ratio. The larger pool of alpha-macroglobulin acts as a reservoir inhibitor. Kane, K.K., Ann. Clin. Lab. Sci. 14:443-449 (1984). Thus, clot lysis by the administration of t-PA is limited by the rapid and irreversible inactivation of plasmin by plasmin inhibitors.

Alpha-2-antiplasmin has three functional domains: the reactive site for plasmin, the plasmin-(ogen) or LBS-binding site [complementary to the LBS (lysine-binding site) of plasmin(ogen)], and the cross-linking site for fibrin. Mimuro, J., et al., Blood 69:446-453 (1987). Mimuro et al. disclose antibodies to alpha-2-antiplasmin, one of which (JPTI-1) was specific to the reactive site of alpha-2-antiplasmin and prevented formation of alpha-2-antiplasmin-plasmin complexes, thereby inhibiting antiplasmin activity. However, Mimuro et al. do not teach administration of the JPTI-1 antibody to enhance clot lysis. Other antibodies specific for alpha-2-antiplasmin are taught by Plow, E.F., et al., J. Biol. Chem. 255:2902-2906 (1980); Wimen, B., et al., Scan. J. Clin. Lab. Invest. 43:27-33 (1983); Hattey, E., et al., Thromb. Res. 45:485-495 (1987); Collen, U.S. Patent No. 4,346,029 (1980); and Collen, U.S. Patent No. 4,198,335 (1980).

Kumada et al., Thromb. Res. 36, 153-163, 1984, disclose the use of polyclonal antibodies to induce α-2-plasmin inhibitor-deficiency in rats by immuno-depletion. Depletion of α-2-plasmin inhibitor resulted in a marked acceleration of urokinase-induced plasma clot lysis, but also in a mild bleeding tendency at the sites of venipuncture.

D. Summary

In summary, a substantial goal of therapies aimed at treating myocardial infarction involves limiting necrosis by permitting early reperfusion and by preventing reocclusion. At present, this goal is partially achieved through the administration of thrombolytic agents capable of dissolving the potentially life-threatening fibrin-platelet clots. The potential benefit of employing such agents is, however, significantly offset by their lack of fibrin specificity (as in the case of streptokinase and urokinase), or by their relatively short biological half-life caused by plasmin inhibitors (which may result in reformation of the fibrin clot, and the accompanying reocclusion of the affected blood vessels). Hence, a need exists for an improvement in thrombolytic therapy which enhances clot lysis, while minimizing fibrinogen breakdown and preventing reocclusion of the affected coronary artery.

## SUMMARY OF THE INVENTION

The present invention provides an improved thrombolytic therapy for the treatment of myocardial infarction, and blood clots within patients. The invention also relates to a method of treating myocardial infarction and blood clots comprising administration a hapten-binding molecule capable of preventing the inhibition of plasmin in an amount sufficient to prevent such inhibition.

The invention also provides a method of treatment for myocardial infarction which comprises coadministrating to a patient in need of such treatment:

(a) a hapten-binding molecule capable of preventing the inhibition of plasmin by a plasmin inhibitor in an amount sufficient to prevent such inhibition; and
(b) a thrombolytic agent, in an amount sufficient to either (i) dissolve a fibrin-platelet clot or (ii) inhibit the formation of a fibrin-platelet clot; wherein the hapten-binding molecule (a) is different from the thrombolytic agent (b).

The invention additionally pertains to a kit useful for carrying out the above method being compartmentalized in close confinement to receive two or more container means therein, which comprises;

(1) a first container containing a therapeutically effective amount of the hapten-binding molecule (a); and
(2) a second container containing a therapeutically effective amount of the thrombolytic agent (b).

## DESCRIPTION OF THE FIGURES

Figure 1 depicts a graph of the percent inhibition of antiplasmin as a function of RWR concentration.

Figure 2 depicts a graph of the percent clot lysis by plasmin as a function of RWR concentration.

Figure 3 depicts a graph of the percent clot lysis by t-PA as a function of RWR concentration.

Figure 4 depicts a graph of the percent clot lysis, wherein RWR is added before or after clotting, as a function of t-PA concentration.

Figure 5 depicts a graph showing the synergistic effects of t-PA and RWR on plasma clot lysis.

Figure 6 depicts and isobologram showing the effects of combinations of RWR and t-PA on plasma clot lysis.

## DESCRIPTION OF THE PREFERRED EMBODIMENTS

The present invention is directed to a method for treating myocardial infarction and blood clots within a patient comprising administering a hapten-binding molecule capable of preventing the inhibition of plasmin, in an amount sufficient to prevent such inhibition.

The present invention also involves a treatment for myocardial infarction and blood clots within a patient which comprises coadministering a hapten-binding molecule and a thrombolytic agent to a recipient patient. A "hapten-binding molecule" is meant to refer to any molecule capable of binding to a plasmin inhibitor. Such molecules may include antibodies, antibody fragments (such as, for example, F(ab')$_2$ or F(ab) molecules), as well as any ligand capable of binding to a plasmin inhibitor.

Blood clots which may be treated according to the methods of the invention include, but are not limited to pulmonary thromboembolism, deep venous thrombosis, cerebral embolism, renal vein and peripheral arterial thrombosis, and the like.

By the term "co-administration" is intended that each of the hapten-binding molecule and thrombolytic agent will be administered during a time frame wherein the respective periods of pharmacological activity overlap. The two agents may be administered simultaneously or sequentially.

The hapten-binding molecules of the present invention may be monoclonal antibodies or fragments thereof. It is preferable to employ the F(ab')$_2$ fragment of such an antibody for this purpose, in order to minimize any immunological reaction caused by the Fc portion of the immunoglobulin. Procedures for preparing monoclonal antibodies are disclosed by Kaprowski, H. et al. (United States Patent No. 4,172,124); and Kohler et al. (Nature 256:495-497 (1975)). The preparation of monoclonal antibodies capable of preventing the inhibition of plasmin are taught by Mimuro, J., et al., Blood 69:446-453 (1987) and described in the examples section of the present application.

As used herein, a "hapten" is a molecule capable of being bound by an antibody. In order to be used in accordance with the present invention, the hapten-binding molecule must be capable of binding to a plasmin inhibitor and thereby prevent such an inhibitor from forming inhibitor-plasmin complexes. Although any such hapten-binding molecule may be employed in accordance with the present invention, it is preferable to employ a hapten-binding molecule which is capable of binding to the plasmin binding site of alpha-2-antiplasmin. An especially preferred monoclonal antibody for this purpose is the antibody RWR described more fully below.

The terms "thrombolytic agent" are meant to refer to any agent capable of either dissolving a fibrin-platelet clot, or inhibiting the formation of such a clot. Examples of thrombolytic agents include streptokinase, prourokinase, urokinase, and tissue-type plasminogen activator. Use of t-PA for these purposes is especially preferred. Although natural t-PA may be employed, it is preferable to employ recombinant t-PA. The invention may additionally employ hybrids, physiologically active fragments or mutant forms of the above thrombolytic agents. The term "tissue-type plasminogen activator" as used herein is intended to include such hybrids, fragments and mutants, as well as both naturally derived and recombinantly derived tissue-type plasminogen activator.

In one embodiment, the hapten-binding molecule and the thrombolytic agent of the present invention are intended to be co-administered to the recipient. It is preferable to provide the hapten-binding molecule to the patient prior to the administration of the thrombolytic agent. It is most preferable to provide the hapten-binding molecule 45 minutes, preferably 30 minutes, prior to the administration of the thrombolytic agent.

The hapten-binding molecule of the present invention is provided for the purpose of preventing the inhibition of plasmin by a plasmin inhibitor. Unexpectedly, it has been discovered that coadministration of the hapten-binding molecule together with a thrombolytic agent causes a synergistic effect, and thereby enhances clot lyses to a greater extent than would be expected if the effects of hapten-binding molecule administration and thrombolytic agent administration was merely additive.

When used alone, an amount of hapten-binding molecule capable of preventing inhibition of plasmin and thereby enhancing clot lysis when provided to a patient is a "therapeutically effective" amount. In order to enhance clot lysis and prevent clot reformation, it is desirable to provide between 3 to 100 nmole of hapten-binding molecule per kilogram of patient weight. This dosage may be administered, in one embodiment, over a period of between 60 to 480 minutes, by continual intravenous infusion at a rate of 0.10 - 1.0 mg/kg min. Alternatively, it is possible to provide the hapten-binding molecule in an intravenously injectable bolus at a dose of between 3 to 100 nmole/kg, and most preferably between 3 to 6 nmole (of hapten-binding molecule) per kilogram of patient weight. If the hapten-binding molecule is provided in this manner, a single bolus is sufficient to prevent potential clot reformation. The hapten-binding molecule of the present invention may be dissolved in any physiologically tolerated liquid in order to prepare an injectable bolus. It is preferable to prepare such a bolus by dissolving the hapten-binding molecule in normal saline.

When the hapten-binding molecule capable of preventing inhibition of plasmin is co-administered with a thrombolytic agent, it is desirable to provide 3 to 6 nmole of hapten-binding molecule per kilogram of patient weight. This dosage may be administered, in one embodiment, over a period of 60 to 480 minutes, by continuous intravenous infusion. Alternatively, it is possible to provide the hapten-binding molecule in an intravenously injectable bolus at a dose of between 3 to 6 nmole/kg, and most preferably between 1 to 3 nmole/kg of patient weight. An amount of thrombolytic agent capable of causing such lysis is a "therapeutically effective" amount. The thrombolytic agent of the present invention is preferably provided at a dose of between 0.5 to 1.0 mg per kg of patient weight. In one embodiment, the thrombolytic agent is provided over a prolonged period (i.e., from about 180 to about 1440 minutes). In a preferred embodiment, the thrombolytic agent of the present invention is provided as an intravenously injected bolus containing between 0.5 to 1.0 mg/kg, and most preferably between 0.5 to 0.75 mg/kg. The thrombolytic agent of the present invention may be dissolved in any physiologically tolerated liquid in order to prepare an injectable bolus. It is, however, preferable to prepare such a bolus by dissolving the thrombolytic agent in normal saline.

A patient treated according to the preferred embodiment will, therefore, receive an intravenously injected bolus of the hapten-binding molecule in combination with an intravenously injected bolus of the thrombolytic agent. This preferred treatment minimizes the amount of t-PA required for thrombolysis, thus reducing the extent of fibrinogen breakdown and lessening any tendency for general hemorrhage. Importantly, the use of the preferred treatment results in the dissolution of the occluding thrombus at a rate which greatly exceeds the rate of thrombus dissolution when either the hapten-binding molecule or the thrombolytic agent is provided by infusion. Additionally, the risk of reocclusion is substantially reduced.

These unexpected findings are important because it had previously not been possible to accelerate the rate of clot lysis without increasing the tendency to hemorrhage. The preferred embodiment, therefore, provides a method of treatment in which the administration of a bolus of a hapten-binding molecule in combination with the administration of a bolus of a thrombolytic agent are capable of dissolving an occluding thrombus at a faster rate than can be obtained when either compound is administered alone. Moreover, the preferred embodiment accomplishes this goal while minimizing both fibrinogen breakdown and the risk of reocclusion.

As would be apparent to one of ordinary skill in the art, the required dosage of the anti-hapten binding molecule or thrombolytic agent will depend upon the severity of the condition of the patient, and upon such criteria as the patient's height, weight, sex, age, and medical history.

The hapten-binding molecule or thrombolytic agent of the present invention can be formulated according to known methods to prepare pharmaceutically useful compositions, such as by admixture with a pharmaceutically acceptable carrier vehicle. Suitable vehicles and their formulation are described, for example, in Remington's Pharmaceutical Sciences (16th Ed., Osol, A. (ed.), Mack, Easton PA (1980)). In order to form a pharmaceutically acceptable composition suitable for effective administration, such compositions will contain an effective amount of the hapten-binding molecule or, thrombolytic agent, either alone, or with a suitable amount of carrier vehicle.

Additional pharmaceutical methods may be employed to control the duration of action. Controlled release preparations may be achieved by the use of polymers to complex or absorb the hapten-binding molecule or thrombolytic agents of the present invention. The controlled delivery may be exercised by selecting appropriate macromolecules (for example, polyesters, polyamino acids, polyvinyl pyrrolidone, ethylenevinylacetate, methylcellulose, carboxymethylcellulose, or protamine sulfate). The rate of drug release may also be controlled by altering the concentration of such macromolecules. Another possible method for controlling the duration of action comprises incorporating the therapeutic agents into particles of a polymeric substance such as polyesters, polyamino acids, hydrogels, poly(lactic acid) or ethylene vinylacetate copolymers. Alternatively, it is possible to entrap the therapeutic agents in microcapsules prepared, for example, by coacervation techniques or by interfacial polymerization, for example, by the use of hydroxymethylcellulose or gelatin-microcapsules or poly(methylmethacrylate) microcapsules, respectively, or in a colloid drug delivery system, for example, liposomes, albumin microspheres, microemulsions, nanoparticles, nanocapsules, or in macroemulsions. Such teachings are disclosed in Remington's Pharmaceutical Sciences (1980).

The thrombolytic agent or hapten-binding molecule may be provided to a patient by means well known in the art. Such means of introduction include oral means, intranasal means, subcutaneous means, intramuscular means, intravenous means, intra-arterial means, or parenteral means. In the most preferred method of treatment for myocardial infarction, a patient is provided with a bolus (intravenously injected) containing between 0.5 to 1.0 mg/kg.

Having now generally described this invention, the same will be better understood by reference to certain specific examples which are included herein for purposes of illustration only, and are not intended to be limiting of the invention, unless specified.

EXAMPLE 1

Preparation of an Antibody Directed to Alpha-2-antiplasmin

A. Materials

Aprotinin and Protein-A Sepharose were purchased from Sigma (St. Louis, MO). S2251 (H-D-Valyl-L-leucyl-L-lysine-p-nitroanilide dihydrochloride) was obtained from Helena Labs (Beaumont, TX). Human alpha-2-antiplasmin (a2AP) was obtained from American Diagnostica, Inc. (Greenwich, CT). PD-10 columns were purchased from Pharmacia (Uppsala, Sweden). Microtiter plates were obtained from Becton Dickinson Labware (Oxnard, CA). Affinity purified goat anti-mouse (Fab')2 IgG was obtained from Cappel Labs (Cochranville, PA). A murine antibody isotyping kit was purchased from Boehringer Mannheim (Indianapolis, IN).

B. Preparation of Murine Monoclonal Antibodies Specific for Alpha-2-antiplasmin

A/J mice were immunized i.p. and s.c. with 10 $\mu$g of human a2AP in Complete Freund's Adjuvant. The mice were boosted 1 month later with 2 $\mu$g of protein in Incomplete Freund's Adjuvant. Two months after initial immunization, the mice were bled from a tail vein and the titres of antibody were determined. The mouse showing the highest titre was hyperimmunized with 5 $\mu$g i.v. (aqueous) and 10 $\mu$g i.p. of a2AP in Incomplete Freund's Adjuvant four days prior, and 5 $\mu$g (aqueous) i.v. 3 days prior to fusion. Splenocytes and murine SP2/0 cells were fused as described (Kohler et al., supra). Hybridomas were selected by a solid phase radioimmunoassay for a2AP immobilized in wells of poly-vinyl chloride microtiter plates. Specifically bound antibody was detected using affinity purified, goat anti-mouse F(ab')2 IgG. Twenty-one hybridomas specifically bound antigen. These hybridomas were subcloned to monoclonality by limiting dilution. Seven hybridomas were expanded into ascites using conventional techniques. Antibody was isolated from filtered ascites using affinity chromatography with Protein-A sepharose. Isotyping was performed using a rabbit anti-mouse, anti-idiotypic antibodies coupled to peroxidase.

One particular hybridoma which produced an antibody (RWR) specific for alpha-2-antiplasmin of the isotype IgGl-K.

EXAMPLE 2

Inhibition of Alpha-2-antiplasmin by RWR

A. Materials and Methods

Iodination of proteins was performed using the Iodogen method. Fraker, P.J. and Speck, J.C., Biochem. Biophys. Res. Comm. 80:849-857 (1978). The specific activity of RWR was 20.0 $\mu$Ci/$\mu$g.

B. Inhibition Studies

Inhibition studies were of several types. First, a chromogenic substrate assay was used to test the ability of RWR to inhibit the inactivation of plasmin by antiplamin. Using a previously described assay (Wiman, B. Meth. Enzymol. 80:1395-1403 (1981)) format, a standard curve was constructed for antiplasmin activity as described. Antiplasmin (0 to 25 nM) was mixed with 0.3 mM S2251 and plasmin (100 nM) in 0.1 M, pH 7.3 phosphate buffer to a total of 1000 ul. The rate of change of optical density at 405 nanometers was automatically recorded every 10 seconds on a Hewlett-Packard 8451A spectrophotometer. A standard curve was constructed which related antiplasmin concentration to the rate of product formation in a linear fashion (r = 0.98). Next, antiplasmin (25 nM) was mixed with RWR (0.875 to 350 nM) and incubated with 0.3mM S2251 in 0.1 M, pH 7.3 phosphate buffer for 2 hours at 25°C. Plasmin (100 nM) was quickly added and the optical density recorded. The rate of change of optical density was used to calculate the amount of inhibition of antiplasmin activity by a given level of antibody.

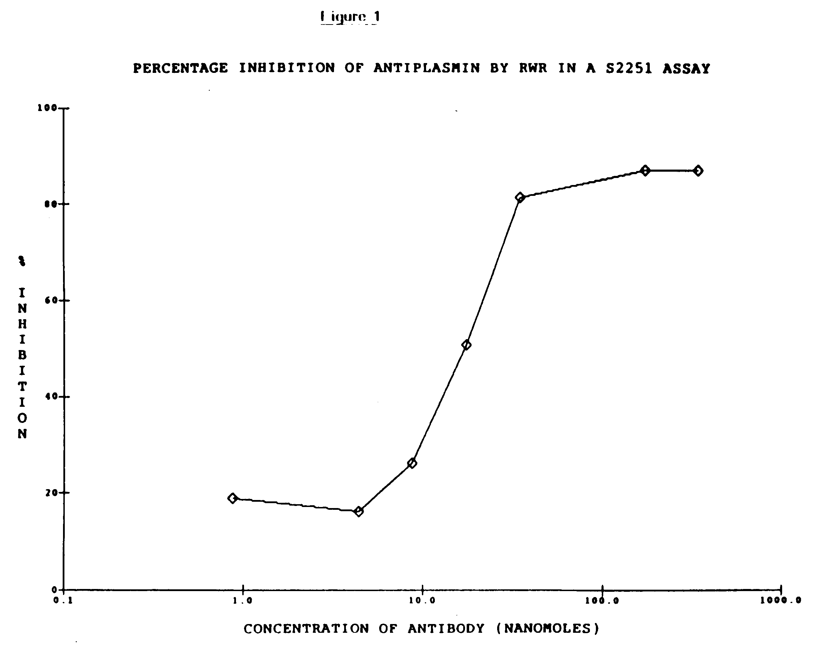

As shown in Figure 1, 55% inhibition of alpha-2-antiplasmin occurred at an antibody concentration of 20 nmole. Immunoblotting under nondenaturing conditions showed that RWR binds the 70,000 Mr species of a2AP and not the 55,000 Mr, non-plasminogen binding form. In addition, RWR did not appreciably bind to denatured or reduced forms of a2AP.

EXAMPLE 3

Enhancement of Clot Lysis with RWR or t-PA

Fresh-frozen, pooled (8 random donors), human plasma was used. To make clots, plasma was mixed with small amounts of Ibrin. In 75 x 12 mm test tubes, 25 $\mu$l of plasma was vortexed with 25 $\mu$l

solution of bovine thrombin (1 U/ml) and calcium chloride (4 mM) in 50 mM Tris-buffered saline pH 7.4. Tubes were placed in a 37°C oscillating water bath to facilitate clotting. The tubes were then counted in a gamma counter to determine the baseline amount of 125-I labeled fibrin incorporated into the clot. Varying amounts of RWR and plasmin were added to each tube clot and the total volume was brought up to 1 ml with 50 mMole Tris, 0.11 M NaCl, and 2 mMole $CaCl_2$. The percentage lysis was determined by measurement of the release of radiolabeled, soluble fibrin peptide. At the indicated time intervals, the supernatant was sampled without replacement and counted. Experiments were done in triplicate and percentage lysis was calculated using the following formula:

Cumulative percentage lysis at time i = $L_i$ = 100*-($S_i$*$TV_i$/$SV_i$ + $\Sigma_{0max(0,i-1)}S_i$)

where $S_i$ = supernatant counts, $TV_i$ = total volume remaining in the tube at time i, $SV_i$ = volume of supernatant sampled at time i, and $\Sigma\, S_i$ represents the sum of previous supernatant samples taken without replacement.

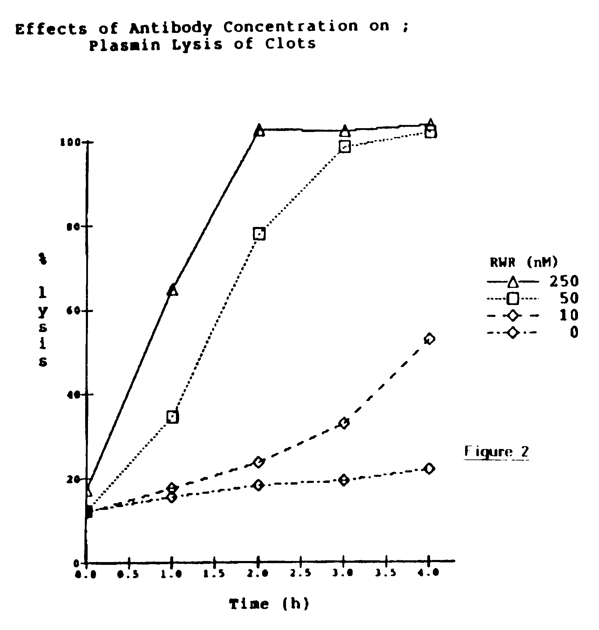

Figure 2 depicts a graph of percent plasma clot lysis as a function of RWR concentration. As can be seen from Figure 2, RWR causes a dose-related significant enhancement of clot lysis by plasmin. Significant clot lysis was observed even at a low concentration of 10 nmole (molar ratio of Mab:a2AP of 0.4:1) over 4 hours. Thus, partial inhibition of a2AP results in a significant increase in fibrinolysis by plasmin. As the concentration of RWR is increased, substantially greater clot lysis was observed.

EXAMPLE 4

Determination of the Synergistic Effect on Clot Lysis by RWR and t-PA

The synergism between t-PA and RWR was studied as previously defined (Loewe, S., Pharmacol. Rev. 9:237-242 (1957); Berenbaum, M.C., Adv. Cancer 35:237-242 (1981)). This analysis requires that the dose-response curves for both agents be constructed (Berenbaum, M.C., J. Theor. Biol. 114:413-421 (1985)). Using this, doses of t-PA and RWR are selected which are isoeffective. Fractional doses of the two agents are then combined and their joint effect on clot lysis is determined. An isobole is constructed which graphs the amount of each agent, alone or in combination, that produces an equivalent amount of lysis in a given period of time.

Alternatively, these types of interactions may be defined algebraically. Given isoeffective doses of each separate agent A & B, the isoeffective fractional doses of each agent in combination ($a_i$ and $b_i$) can be characterized as: synergistic if $a_i$/A + $b_i$/B < 1, additive if ai/A + Bi/B = 1, or antagonistic if $a_i$/A + Bi/B >1.

To determine this empirically, various amounts of t-PA and RWR were added separately and in combination to test tube labeled clots (in triplicate as described below). Then the mean percentage lysis was determined as indicated.

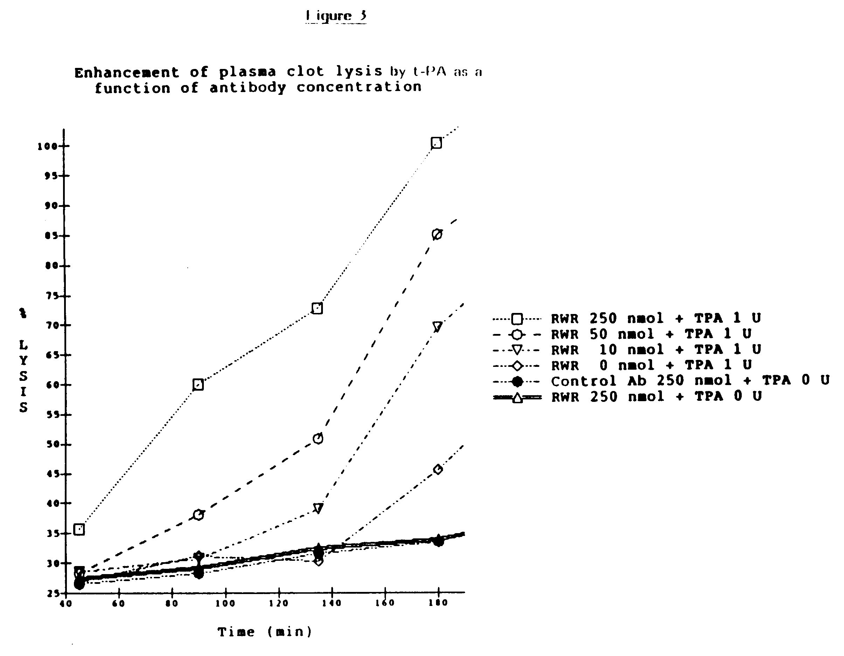

Fresh-frozen plasma was mixed with 125-I labeled fibrinogen and clotted. Then varying amounts of RWR (0 to 250 mmol, final) or ao control (anti-digoxin) antibody (0 or 250 nmol, final) were added to tubes containing 0 or 1 units of t-PA. Tubes were incubated at 37°C and the percentage lysis was determined by the release of radiolabeled fibrin peptides. In control clots, in the time indicated, RWR alone was not different from a control antibody alone--an anti-digoxin Mab 40-160 (Mudgett-Hunter, M., et al., Mol. Immunol. 22:477-488 (1985)) --in the amount of lysis induced without t-PA. However, in the presence of t-PA, RWR shows a dose-related enhancement of clot lysis which greater than that seen in clots tested with t-PA alone. Figure 3 shows the effect of varying concentrations of t-PA and RWR on clot lysis.

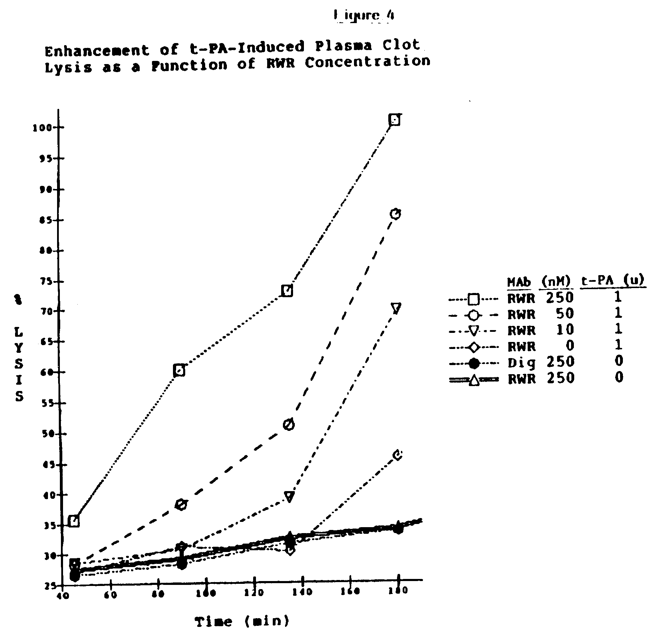

Approximately 30% of antiplasmin is cross-linked to fibrin by Factor 13. If cross-linked antiplasmin is not available to the antibody for binding, this would significantly decrease the antibody's effect on the rate of clot lysis. As such clots formed with antibody already bound to a2AP should lyse more quickly than those to whom the antibody was added after cloning, i.e., after the cross-linking of a2AP to fibrin. To test this hypothesis, the time-related lysis in clots formed after preincubation of the plasma with RWR was compared to that of formed clots to which RWR was subsequently added (Figure 4).

Fresh-frozen plasma was mixed with radiolabeled fibrinogen. Then RWR (25 nmole, final) was mixed with plasma and clotted. In other experiments, RWR was added to already formed plasma clots. Clots were subsequently incubated in buffer for 24 hours at 4°C. Then t-PA (0, 0.1, or 1 u) was added and the clots incubated were at 37°C. The rate of lysis was determined as described above. It can be seen in Figure 4 that there is only a slight increase in the rate of lysis for clots which were exposed to RWR prior to formation. This suggests that antiplasmin is functionally accessible to the MAb in cross-linked fibrin.

In order to determine the effect of combinations of t-PA and RWR on lysis, an isobole was constructed as recommended by previous studies (Loewe, 1957; Berenbaum, 1981). First, isoeffective doses of t-PA and RWR were determined.

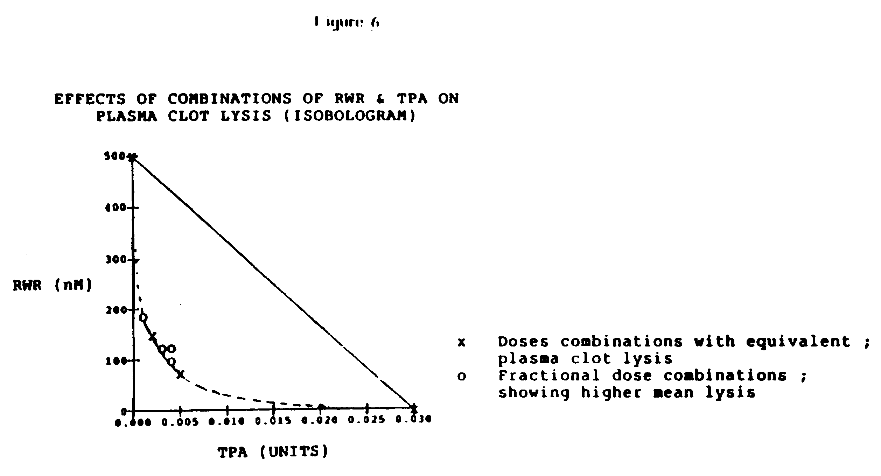

Radiolabeled plasma clots were incubated at 37°C with varying amounts of t-PA (0 to 0.03 units) or RWR (0 to 500 nmol). The percentage lysis at 48 hours was determined (Figure 6). Control clots (without either agent) had 9.36 + 0.38% lysis during this study. In this experiment, 0.3 U of t-PA and 500 nmol RWR produced an equivalent amount of lysis (35.5 + 3.1 vs. 34.3 + 5.1; mean + SEM).

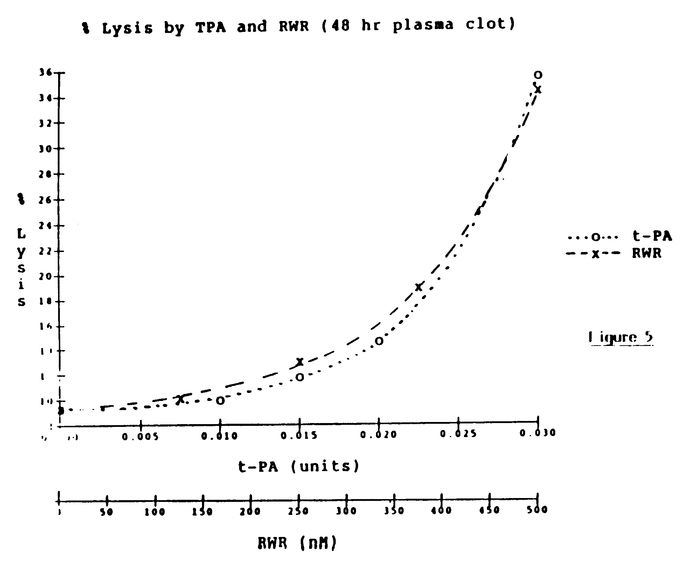

In the same experiment, various empiric, fractional combinations of RWR and t-PA were also tested. Fractional dose combinations of both agents showing equivalent lysis to 500 nmol RWR or 0.03 U of t-PA are plotted (X) on Figure 6. Dose combinations of RWR and t-PA which produced the same amount of lysis (34.2%, 34.9%) as these agents alone are indicated by X. Dose combinations showing higher mean lysis (41.3% to 100%) are denoted by 0. Because the equipotent combinations of t-PA and RWR are a small fraction of each agent alone (i.e., they lie far below the line of additivity indicated by the continuous line in Figure 5), there is evidence of strong synergy between these two agents.

## Claims

**Claims for the following Contracting States :
AT, BE, CH, DE, FR, GB, IT, LI, LU, NL, SE**

1. A composition containing:
   a. a monoclonal antibody, or a fragment thereof, capable of binding to $\alpha$-2-antiplasmin and preventing inactivation of plasmin; and
   b. a thrombolytic agent in an amount sufficient to either (i) dissolve a fibrin-platelet clot or (ii) inhibit the formation of a fibrin-platelet clot, which is different from the antibody or fragment thereof in (a);
   as a combined preparation for simultaneous, separate or sequential use in a method of treating myocardial infarction and/or blood clots.

2. A pharmaceutical composition comprising:
   a. a monoclonal antibody, or a fragment thereof, capable of binding to $\alpha$-2-antiplasmin and preventing inactivation of plasmin; and
   b. a thrombolytic agent in an amount sufficient to either (i) dissolve a fibrin-platelet clot or (ii) inhibit the formation of a fibrin-platelet clot, which is different from the antibody or fragment thereof in (a);
   in admixture with a pharmaceutically acceptable carrier vehicle.

3. A composition as claimed in claim 1 or 2 wherein both the antibody or a fragment thereof (a) and the thrombolytic agent (b) are adapted to be administered to a patient by intravenous infusion.

4. A composition as claimed in claim 1 or 2 wherein both the antibody or a fragment thereof (a) and the thrombolytic agent (b) are adapted to be administered to a patient by an intravenously injected bolus.

5. A composition according to claim 1 or 2 which is adapted to be administered orally.

6. A composition as claimed in any of claims 1 to 5 wherein the fragment of the antibody is an F-(ab')$_2$ fragment.

7. A composition as claimed in any of claims 1 to 6, wherein the thrombolytic agent comprises streptokinase, prourokinase, urokinase and/or, preferably, tissue-type plasminogen activator.

8. The use of:
   a. a monoclonal antibody, or a fragment thereof, capable of binding to $\alpha$-2-antiplasmin and preventing inactivation of plasmin; and
   b. a thrombolytic agent in an amount sufficient to either (i) dissolve a fibrin-platelet clot or (ii) inhibit the formation of a fibrin-platelet clot, which is different from the antibody or fragment thereof in (a);
   in the preparation of a medicament for treating myocardial infarction or blood clots.

9. The use as claimed in claim 8, wherein:
   (1) the antibody or fragment thereof (a) is adapted to be provided to a patient at a dose of between 3 to 6 nmole per kg of patient weight; and
   (2) the thrombolytic agent (b) is adapted to be provided to a patient at a dose of between 0.5 to 1.0 mg per kg of patient weight, and preferably at a dose of between 0.5 to 0.75 mg per kg of patient weight.

10. A medicament kit being compartmentalized in close confinement to receive two or more container means therein, which comprises:
    (1) a first container containing a therapeutically effective amount of a monoclonal antibody, or a fragment thereof, capable of binding to $\alpha$-2-antiplasmin and preventing inactivation of plasmin; and
    (2) a second container containing a therapeutically effective amount of a thrombolytic agent (b).

**11.** A kit according to claim 10 wherein the thrombolytic agent comprises streptokinase, prourokinase, urokinase and/or, preferably, tissue-type plasminogen activator.

**12.** A kit according to claim 10 or 11 wherein the fragment of the antibody is in F(ab')$_2$ fragment.

**Claims for the following Contracting States : ES, GR**

**1.** A process for the preparation of a pharmaceutically acceptable composition, the process comprising admixing:

a. a monoclonal antibody, or a fragment thereof, capable of binding to $\alpha$-2-antiplasmin and preventing inactivation of plasmin;
b. a thrombolytic agent in an amount sufficient to either (i) dissolve a fibrin-platelet clot or (ii) inhibit the formation of a fibrin-platelet clot, which is different from the antibody or fragment thereof in (a);
with a pharmaceutically acceptable carrier vehicle.

**2.** A process as claimed in claim 1 wherein the composition is adapted for both the antibody or a fragment thereof (a) and the thrombolytic agent (b) to be administered to a patient by intravenous infusion.

**3.** A process as claimed in claim 1 wherein the composition is adapted for both the antibody or a fragment thereof (a) and the thrombolytic agent (b) to be administered to a patient by an intravenously injected bolus.

**4.** A process according to claim 1 or 2 wherein the composition is adapted for oral administration.

**5.** A process as claimed in any of claims 1 to 4 wherein the fragment of the antibody is an F-(ab')$_2$ fragment.

**6.** A process as claimed in any of claims 1 to 5, wherein the thrombolytic agent comprises streptokinase, prourokinase, urokinase and/or, preferably, tissue-type plasminogen activator.

**7.** The use of:

a. a monoclonal antibody, or a fragment thereof, capable of binding to $\alpha$-2-antiplasmin and preventing inactivation of plasmin; and

b. a thrombolytic agent in an amount sufficient to either (i) dissolve a fibrin-platelet clot or (ii) inhibit the formation of a fibrin-platelet clot, which is different from the antibody or fragment thereof in (a);

in the preparation of a medicament for treating myocardial infarction or blood clots.

**8.** The use as claimed in claim 7, wherein:

(1) the antibody or fragment thereof (a) is adapted to be provided to a patient at a dose of between 3 to 6 nmole per kg of patient weight; and
(2) the thrombolytic agent (b) is adapted to be provided to a patient at a dose of between 0.5 to 1.0 mg per kg of patient weight, and preferably at a dose of between 0.5 to 0.75 mg per kg of patient weight.

**9.** The use according to claim 7 or 8 wherein the thrombolytic agent comprises streptokinase, prourokinase, urokinase and/or, preferably, tissue-type plasminogen activator.

**Patentansprüche**
**Patentansprüche für folgende Vertragsstaaten : AT, BE, CH, DE, FR, GB, IT, LI, LU, NL, SE**

**1.** Zusammensetzung, enthaltend:

a. einen monoklonalen Antikörper oder ein Fragment davon, welcher(s) in der Lage ist, an $\alpha$-2-Antiplasmin zu binden und die Inaktivierung von Plasmin zu verhindern; und
b. ein thrombolytisches Mittel in einer Menge, welche dazu ausreicht, um entweder (i) ein Fibrin/Blutplättchen-Gerinnsel aufzulösen oder (ii) die Bildung eines Fibrin/Blutplättchen-Gerinnsels zu hemmen, welches Mittel sich vom Antikörper oder dessen Fragment gemäß (a) unterscheidet;
als ein kombiniertes Präparat zur gleichzeitigen, getrennten oder aufeinanderfolgenden Verwendung in einem Verfahren zur Behandlung von Myokardinfarkt und/oder Blutgerinnseln.

**2.** Pharmazeutische Zusammensetzung, bei welcher

a. einen monoklonalen Antikörper oder ein Fragment davon, welcher(s) in der Lage ist, an $\alpha$-2-Antiplasmin zu binden und die Inaktivierung von Plasmin zu verhindern; und
b. ein thrombolytisches Mittel in einer Menge, welche dazu ausreicht, um entweder (i) ein Fibrin/Blutplättchen-Gerinnsel auflösen oder (ii) die Bildung eines Fibrin/Blutplättchen-Gerinnsels zu hemmen, welches Mittel sich vom Antikörper oder dessen Fragment gemäß (a) unterscheidet;
mit einem pharmazeutisch akzeptablen Trägervehikel versetzt ist.

3. Zusammensetzung nach Anspruch 1 oder 2, wobei sowohl der Antikörper oder ein Fragment davon (a) als auch das thrombolytische Mittel (b) zubereitet sind, um einem Patienten durch intravenöse Infusion verabreicht zu werden.

4. Zusammensetzung nach Anspruch 1 oder 2, wobei sowohl der Antikörper oder ein Fragment davon (a) als auch das thrombolytische Mittel (b) zubereitet um einem Patienten mittels eines intravenös injizierten Bolus verabreicht zu werden.

5. Zusammensetzung nach Anspruch 1 oder 2, welche zubereitet ist, um oral verabreicht zu werden.

6. Zusammensetzung nach einem der Ansprüche 1 bis 5, wobei das Fragment des Antikörpers ein F(ab')$_2$-Fragment ist.

7. Zusammensetzung nach einem der Ansprüche 1 bis 6, wobei das thrombolytische Mittel Streptokinase, Prourokinase, Urokinase und/oder vorzugsweise Gewebsplasminogen-Aktivator umfaßt.

8. Verwendung:
a. eines monoklonalen Antikörpers oder eines Fragments davon, welcher(s) in der Lage ist, an $\alpha$-2-Antiplasmin zu binden und die Inaktivierung von Plasmin zu verhindern; und
b. eines thrombolytischen Mittels in einer Menge, welche dazu ausreicht, um entweder (i) ein Fibrin/Blutplättchen-Gerinnsel aufzulösen oder (ii) die Bildung eines Fibrin/Blutplättchen-Gerinnsels zu hemmen, welches Mittel sich vom Antikörper oder dessen Fragment gemäß (a) unterscheidet;
bei der Herstellung eines Medikamentes zur Behandlung von Myokardinfarkt oder Blutgerinnseln.

9. Verwendung nach Anspruch 8, wobei:
(1) der Antikörper oder das Fragment davon (a) zubereitet ist, um einem Patienten in einer Dosierung zwischen 3 und 6 nMol je kg Körpergewicht des Patienten zur Verfügung gestellt zu werden; und
(2) das thrombolytische Mittel (b) zubereitet ist, um einem Patienten in einer Dosierung zwischen 0,5 und 1,0 mg je kg Körpergewicht des Patienten, und vorzugsweise in einer Dosierung zwischen 0,5 und 0,75 mg je kg Körpergewicht des Patienten, zur Verfügung gestellt zu werden.

10. Medikamentensatz, welcher dicht verschlossen in Fächer geteilt ist, um darin zwei oder mehrere Behältermittel aufzunehmen, welche:
(1) einen ersten Behälter, welcher eine therapeutisch wirksame Menge eines monoklonalen Antikörpers oder ein Fragment davon enthält, welcher(s) in der Lage ist, an $\alpha$-2-Antiplasmin zu binden und die Inaktivierung von Plasmin zu verhindern; und
(2) einen zweiten Behälter, welcher eine therapeutisch wirksame Menge eines thrombolyischen Mittels (b) enthält;
umfassen.

11. Satz nach Anspruch 10, wobei das thrombolytische Mittel Streptokinase, Prourokinase, Urokinase und/oder vorzugsweise Gewebsplasminogen-Antivator umfaßt.

12. Satz nach Anspruch 10 oder 11, wobei das Fragment des Antikörpers ein F(ab')$_2$-Fragment ist.

**Patentansprüche für folgende Vertragsstaaten : ES, GR**

1. Verfahren zur Herstellung einer pharmazeutisch akzeptablen Zusammensetzung, wobei das Verfahren das Versetzen:
a. eines monoklonalen Antikörpers oder eines Fragments davon, welcher(s) in der Lage ist, an $\alpha$-2-Antiplasmin zu binden und die Inaktivierung von Plasmin zu verhindern;
b. eines thrombolytischen Mittels in einer Menge, welche dazu ausreicht, um entweder (i) ein Fibrin/Blutplättchen-Gerinnsel aufzulösen oder (ii) die Bildung eines Fibrin/Blutplättchen-Gerinnsels zu hemmen, welches Mittel sich vom Antikörper oder dessen Fragment gemäß (a) unterscheidet;
mit einem pharmazeutisch akzeptablen Trägervehikel umfaßt.

2. Verfahren nach Anspruch 1, wobei die Zusammensetzung derart ausgebildet ist, daß sowohl der Antikörper oder ein Fragment davon (a) als auch das thrombolytische Mittel (b) einem Patienten durch intravenöse Infusion verabreicht werden.

3. Verfahren nach Anspruch 1, wobei die Zusammensetzung derart zubereitet ist, daß sowohl der Antikörper oder ein Fragment davon (a) als auch das thrombolytische Mittel (b) einem Patienten mittels eines intravenös injizierten Bolus verabreicht werden.

4. Verfahren nach Anspruch 1 oder 2, wobei die Zusammensetzung zubereitet ist, um oral verabreicht zu werden.

5. Verfahren nach einem der Ansprüche 1 bis 4, wobei das Fragment des Antikörpers ein F-(ab')$_2$-Fragment ist

6. Verfahren nach einem der Ansprüche 1 bis 5, wobei das thrombolytische Mittel Streptokinase, Prourokinase, Urokinase und/oder vorzugsweise Gewebsplasminogen-Aktivator enthält.

7. Verwendung:
a. eines monoklonalen Antikörpers oder eines Fragments davon, welcher(s) in der Lage ist, an $\alpha$-2-Antiplasmin zu binden und die Inaktivierung von Plasmin zu verhindern; und
b. eines thrombolytischen Mittels in einer Menge, welche dazu ausreicht, um entweder (i) ein Fibrin/Blutplättchen-Gerinnsel aufzulösen oder (ii) die Bildung eines Fibrin/Blutplättchen-Gerinnsels zu hemmen, welches Mittel sich vom Antikörper oder dessen Fragment gemäß (a) unterscheidet;
bei der Herstellung eines Medikaments zur Behandlung von Myokardinfarkt oder Blutgerinnseln.

8. Verwendung nach Anspruch 7, wobei:
(1) der Antikörper oder das Fragment davon (a) zubereitet ist, um einem Patienten in einer Dosierung zwischen 3 und 6 nMol je kg Körpergewicht des Patienten zur Verfügung gestellt zu werden; und
(2) das thrombolytische Mittel (b) zubereitet ist, um einem Patienten in einer Dosierung zwischen 0,5 und 1,0 mg je kg Körpergewicht des Patienten, und vorzugsweise in einer Dosierung zwischen 0,5 und 0,75 mg je kg Körpergewicht des Patienten, zur Verfügung gestellt zu werden.

9. Verwendung nach Anspruch 7 oder 8, wobei das thrombolytische Mittel Streptokinase, Prourokinase, Urokinase und/oder vorzugsweise Gewebsplasminogen-Aktivator umfaßt.

**Revendications**

**Revendications pour les Etats contractants suivants : AT, BE, CH, DE, FR, GB, IT, LI, LU, NL, SE**

1. Composition contenant :
a. un anticorps monoclonal ou un fragment de celui-ci, capable de lier l'alpha-2-antiplasmine et de prévenir l'inactivation de la plasmine, et
b. un agent thrombolytique en une quantité suffisante pour :
(i) dissoudre un caillot de fibrine-plaquettes, ou
(ii) inhiber la formation d'un caillot de fibrine-plaquettes,
qui est différent de l'anticorps ou du fragment de celui-ci en (a);
sous forme d'une préparation combinée pour une utilisation simultanée, séparée ou séquentielle dans un procédé de traitement de l'infarctus du myocarde et/ou de caillots sanguins.

2. Composition pharmaceutique comprenant :
a. un anticorps monoclonal ou un fragment de celui-ci, capable de lier l'alpha-2-antiplasmine et de prévenir l'inactivation de la plasmine, et
b. un agent thrombolytique en une quantité suffisante pour :
(i) dissoudre un caillot de fibrine-plaquettes, ou
(ii) inhiber la formation d'un caillot de fibrine-plaquettes,
qui est différent de l'anticorps ou du fragment de celui-ci en (a);
en mélange avec un véhicule support pharmaceutiquement acceptable.

3. Composition suivant la revendication 1 ou 2, dans laquelle l'anticorps ou le fragment de celui-ci (a) et l'agent thrombolytique (b) sont appropriés pour être administrés à un patient par injection intraveineuse.

4. Composition suivant la revendication 1 ou 2, dans laquelle l'anticorps ou le fragment de celui-ci (a) et l'agent thrombolytique (b) sont appropriés pour être administrés à un patient par injection intraveineuse à la seringue.

5. Composition suivant la revendication 1 ou 2, qui est appropriée à être administrée oralement.

**6.** Composition suivant l'une quelconque des revendications 1 à 5, dans laquelle le fragment de l'anticorps est un fragment F(ab')$_2$.

**7.** Composition suivant l'une quelconque des revendications 1 à 6, dans laquelle l'agent thrombolytique peut être la streptokinase, la prourokinase, l'urokinase et/ou, de préférence, un activateur de plasminogène de type tissulaire.

**8.** Utilisation de :
   a. un anticorps monoclonal ou un fragment de celui-ci, capable de lier l'alpha-2-antiplasmine et de prévenir l'inactivation de la plasmine, et
   b. un agent thrombolytique en une quantité suffisante pour :
      (i) dissoudre un caillot de fibrine-plaquettes, ou
      (ii) inhiber la formation d'un caillot de fibrine-plaquettes,
      qui est différent de l'anticorps ou du fragment de celui-ci en (a);
      dans la préparation d'un médicament pour traiter l'infarctus du myocarde ou des caillots sanguins.

**9.** Utilisation suivant la revendication 8, dans laquelle :
   (1) l'anticorps ou le fragment de celui-ci (a) est approprié pour être donné à un patient à une dose comprise entre 3 et 6 nmoles par kg de poids de patient, et
   (2) l'agent thrombolytique (b) est approprié pour être donné à un patient à une dose comprise entre 0,5 et 1,0 mg par kg de poids du patient et, de préférence, à une dose comprise entre 0,5 et 0,75 mg par kg de poids de patient.

**10.** Equipement médicamenteux compartimenté pour contenir deux ou plusieurs dispositifs récepteurs, qui comprend :
   (1) un premier récipient contenant une quantité thérapeutiquement efficace d'un anticorps monoclonal ou d'un fragment de celui-ci, capable de lier l'alpha-2-antiplasmine et de prévenir l'inactivation de la plasmine, et
   (2) un deuxième récipient contenant une quantité thérapeutiquement efficace d'un agent thrombolytique (b).

**11.** Equipement suivant la revendication 10, dans lequel l'agent thrombolytique peut être la streptokinase, la prourokinase, l'urokinase et/ou, de préférence, un activateur de plasminogène de type tissulaire.

**12.** Equipement suivant la revendication 10 ou 11, dans lequel le fragment de l'anticorps est un fragment F(ab')$_2$.

**Revendications pour les Etats contractants suivants : ES, GR**

**1.** Procédé de préparation d'une composition pharmaceutiquement acceptable, le procédé comprenant le mélange de :
   a. un anticorps monoclonal ou un fragment de celui-ci, capable de lier l'alpha-2-antiplasmine et de prévenir l'inactivation de la plasmine, et
   b. un agent thrombolytique en une quantité suffisante pour :
      (i) dissoudre un caillot de fibrine-plaquettes, ou
      (ii) inhiber la formation d'un caillot de fibrine-plaquettes,
      qui est différent de l'anticorps ou du fragment de celui-ci en (a);
      avec un véhicule support pharmaceutiquement acceptable.

**2.** Procédé suivant la revendication 1, dans lequel la composition est appropriée, pour l'anticorps ou le fragment de celui-ci (a) et pour l'agent thrombolytique (b), pour être administrée à un patient par injection intraveineuse.

**3.** Procédé suivant la revendication 1, dans lequel la composition est appropriée, pour l'anticorps ou le fragment de celui-ci (a) et pour l'agent thrombolytique (b), pour être administrée à un patient par injection intraveineuse à la seringue.

**4.** Procédé suivant la revendication 1 ou 2, dans lequel la composition est appropriée pour une administration orale.

**5.** Procédé suivant l'une quelconque des revendications 1 à 4, dans lequel le fragment de l'anticorps est un fragment F(ab')$_2$.

**6.** Procédé suivant l'une quelconque des revendications 1 à 5, dans lequel l'agent thrombolytique peut être la streptokinase, la prourokinase, l'urokinase et/ou, de préférence, un activateur de plasminogène de type tissulaire.

**7.** Utilisation de :
   a. un anticorps monoclonal ou un fragment de celui-ci, capable de lier l'alpha-2-antiplasmine et de prévenir l'inactivation de la plasmine, et

b. un agent thrombolytique en une quantité suffisante pour :

(i) dissoudre un caillot de fibrine-plaquettes, ou

(ii) inhiber la formation d'un caillot de fibrine-plaquettes,

qui est différent de l'anticorps ou du fragment de celui-ci en (a);

dans la préparation d'un médicament pour traiter l'infarctus du myocarde ou des caillots sanguins.

8. Utilisation suivant la revendication 7, dans laquelle :

(1) l'anticorps ou le fragment de celui-ci (a) est approprié pour être donné à un patient à une dose comprise entre 3 et 6 nmoles par kg de poids de patient, et

(2) l'agent thrombolytique (b) est approprié pour être donné à un patient à une dose comprise entre 0,5 et 1,0 mg par kg de poids du patient et, de préférence, à une dose comprise entre 0,5 et 0,75 mg par kg de poids du patient.

9. Utilisation suivant la revendication 7 ou 8, dans laquelle l'agent thrombolytique peut être la streptokinase, la prourokinase, l'urokinase et/ou, de préférence, un activateur de plasminogène de type tissulaire.

Figure 1

PERCENTAGE INHIBITION OF ANTIPLASMIN BY RWR IN A S2251 ASSAY

EP 0 336 693 B1

# Effects of Antibody Concentration on ; Plasmin Lysis of Clots

RWR (nM)
—△— 250
···□··· 50
— ◇ — 10
—·◇·— 0

% lysis

Time (h)

Figure 2

Figure 3

**Enhancement of plasma clot lysis by t-PA as a
function of antibody concentration**

Legend:
- RWR 250 nmol + TPA 1 U
- RWR 50 nmol + TPA 1 U
- RWR 10 nmol + TPA 1 U
- RWR 0 nmol + TPA 1 U
- Control Ab 250 nmol + TPA 0 U
- RWR 250 nmol + TPA 0 U

Time (min)

EP 0 336 693 B1

Figure 4

**Enhancement of t-PA-Induced Plasma Clot
Lysis as a Function of RWR Concentration**

| | MAb | (nM) | t-PA (u) |
|---|---|---|---|
| ----□---- | RWR | 250 | 1 |
| - -○- - | RWR | 50 | 1 |
| - -▽- - | RWR | 10 | 1 |
| ----◇---- | RWR | 0 | 1 |
| ----●---- | Dig | 250 | 0 |
| ═△═ | RWR | 250 | 0 |

% LYSIS

Time (min)

EP 0 336 693 B1

# % Lysis by TPA and RWR (48 hr plasma clot)

Figure 5

Figure 6

**EFFECTS OF COMBINATIONS OF RWR & TPA ON
PLASMA CLOT LYSIS (ISOBOLOGRAM)**

x   Doses combinations with equivalent ;
plasma clot lysis

o   Fractional dose combinations ;
showing higher mean lysis

EP 0 336 693 B1